Europäisches Patentamt

European Patent Office

Office européen des brevets

(19)

(11) Publication number: 0 424 586 A2

# EUROPEAN PATENT APPLICATION

(21) Application number: 89312157.4

(22) Date of filing: 22.11.89

(51) Int. Cl.5: G01N 33/543, G01N 33/553, G01N 33/535, G01N 33/53, C12Q 1/42

(30) Priority: 26.10.89 GB 8923868

(43) Date of publication of application: 02.05.91 Bulletin 91/18

(84) Designated Contracting States: AT BE CH DE ES FR GB GR IT LI LU NL SE

(71) Applicant: IMMUNOSENS SPA
Via Michelle Mattei 17
Benevento(IT)

(72) Inventor: McNeil, Calum Jack
201 Osborne Road
Jesmond Newcastle Upon Tyne(GB)
Inventor: Bannister, Joseph
43 Elms Drive
Old Marston Oxford(GB)
Inventor: Athey, Dale
17 Coast View
Swarland Morpeth Northumberland(GB)

(74) Representative: Browne, Robin Forsythe et al
Urquhart-Dykes & Lord Tower House Merrion Way
Leeds LS2 8PA West Yorkshire(GB)

(54) Method and apparatus for electrochemical immunoassay.

(57) A method of eletrochemical alkaline phosphatase immunoassay comprises the steps of:
contacting the alkaline phosphatase with 1-naphthyl phosphate, allowing the phosphatase to hydrolyse the 1-naphthyl phosphate to form 1-naphthol and detecting the electrochemical oxidation potential of said 1-naphthol using an electrode comprising resin bonded particles of carbon having a particle size of 3 to 50 nm the particles carrying a platinum group metal.

EP 0 424 586 A2

## METHOD AND APPARATUS FOR ELECTROCHEMICAL IMMUNOASSAY

This invention relates to a method and apparatus for electrochemical immunoassay. Immunoassay techniques are increasingly used in medicine for detection of a wide range of substances, for example hormones, and are also used in diagnosis, for example in animal husbandry and food chemistry.

Early immunoassay techniques have involved radio labelling or spectrophotometry. Enzyme immunoassays are preferred due to reduction in health hazards, low cost, simplification of equipment, applicability of automation and stability of the reagents employed. In enzyme linked immunosorbent assay (ELISA) enzymes such as horseradish peroxidase, alkaline phosphatase and beta-galactosidase have been coupled to the analyte, so that the latter can be determined from the activity of the enzyme. Spectrophotometric methods have been used to measure concentration of a chromophore resultant from reaction of the enzyme. Use of colour forming substrates has drawbacks, for example the chromophore may be unstable, the length of time required to obtain results may be too long and the linear dynamic range of the assay procedure may be limited by the expensive optical instrumentation.

Electrochemical detection can be used as an alternative to spectrophotometry. A number of rapid, simple enzyme electrodes have been developed for measurement of substances of clinical importance. The combination of substrate specificity of an enzyme with the sensitivity of electrochemical methods has been applied to design of a number of electrochemical immunoassays, some of which have similar intrinsic sensitivity to radio immunoassay. Electrochemical methods have been most successfully applied to determination of the activity of redox enzymes, for example glucose oxidase bound on a stationary support. The label catalyses a redox reaction which may be determined at an electrode surface. The sensitivity of such assays is too low to allow some analytes to be detected in routinely encountered concentrations. This problem can be overcome by use of hydrolytic enzymes which can generate electrochemically active products from electrochemically inactive and thus non-interfering substrates. Use of the enzyme label, alkaline phosphatase, has been disclosed (Analytical Chemistry (1984), 56 , 2355 to 2360) wherein the electroinactive compound phenyl phosphate is converted to the electrochemically active product phenol. Phenol can be detected by its oxidation at + 670mV vs Ag/AgCl. This method is not generally applicable to systems incorporating components which oxidise at this potential, rendering it impractical for use with blood or serum samples.

According to a first aspect of the present invention, a method of electrochemical alkaline phosphatase immunoassay comprises the steps of:

contacting the alkaline phosphatase with a 1-naphthyl phosphate, allowing the phosphatase to hydrolyse the 1-naphthyl phosphate to form 1-naphthol and detecting the electrochemical oxidation potential of said 1-naphthol using an electrode comprising particles of carbon having a particle size of 3 to 50 nm the particles carrying a platinum group metal.

Use of 1-naphthyl phosphate in conjunction with the specified electrode affords particular advantages. The current density obtained at the electrode is unexpectedly higher than may be obtained using conventional electrodes. This provides much higher sensitivity than had been possible hitherto. The redox potential of 1-naphthyl phosphate is lower than those of potentially interfering compounds which may be present in blood or serum samples. In addition use of 1-naphthyl phosphate does not exhibit the drawbacks which may be evident from use of alternative phosphates having low redox potentials. For example, 4-aminophenyl phosphate, in addition to not being commercially available, is extremely unstable, oxidising and polymerising very rapidly at room temperature, leaving polymeric deposits on the electrode. This compound could not be used for prolonged incubation of alkaline phosphatase with a substrate as is required in certain hormone immunoassays.

According to a second aspect of the present invention alkaline phosphatase immunoassay apparatus includes at least one electrochemical cell containing a reference electrode and an electrode comprising carbon particles having a particle size of 3 to 50 nm carrying a platinum group metal.

The carbon particles may be bonded using a resin or other binder. The potential between the electrodes is arranged in use to correspond to the redox potential of the 1-naphthol compound + 300 mV having been found to be suitable. The reference electrode may comprise Ag/AgCl, stainless steel or other conventional reference electrodes.

An array of electrochemical cells may be provided to facilitate multiple simultaneous determinations, for example on a printed circuit board as disclosed in WO86/03837.

Preferred electrodes comprise an electrically conducting porous layer of bonded carbon or graphite particles, a finely divided platinum group metal for example platinum or palladium being intimately mixed with, deposited or absorbed onto the particles prior to bonding for example with a resin to form said layer.

2

Preferred resin binders are fluorocarbon resins especially polytetrafluoroethylene. Alternative electrodes may be made by screen printing using suspensions of the platinised carbon particles in a suitable carrier.

Any suitable carbon powder having a particle size of 30 to 50 nm may be used. Preferred particle sizes are 5 to 30 nm.

Platinum or palladium may be deposited on the carbon particles by vapour phase deposition, electrochemical deposition or preferably simple absorption from colloidal suspension. 1 to 20% , preferably 5 to 15% based on the weight of carbon may conveniently be employed. The platinised or palladised carbon powder may be moulded with the fluorocarbon resin to form a self-supporting porous structure. Alternatively the resin bonded particles may be disposed upon a substrate for example carbon paper, or porous carbon cloth. The manufacture and properties of the preferred electrodes is disclosed in US-A-4044193, US-A-4166143, US-A-4293396 and US-4478696.

The method and apparatus of this invention are readily adapted to immunoassays of clinically important substances coupled to alkaline phosphatase.

The invention is further described by means of example but not in any limitation sense, with reference to the accompanying drawings of which:

Materials and Methods

Alkaline phosphatase (E.C. 3.1.3.1), was obtained from B.C.L.

Optical experiments with paranitrophenol phosphate involved the use of Dynatech microtitration plates and Titertek plate reader.

All substrate and product solutions were prepared freshly to minimise non-enzymatic hydrolysis.

The carbon electrodes were supplied by Prototech Company, Newton, Mass, USA. Glassy carbon electrodes were obtained from Oxford Electrodes.

Buffers

All buffer solutions were prepared using distilled, deionised water.

(i) Tris/HC1 pH 10.2

Tris 100 mM, NaCl 50 mM, MgCl₂, 1mM, ZnSO₄ 0.1 mM.

Stock solutions (a) MgCl₂, (20.3 g/100 mL) 1M, (b) ZnSO₄ (2.875 g/100 mL) 100 mM, (c) Tris-[hydroxymethyl]aminoethane (30.25g/500 mL) 500 mM, (d) NaCl (14.61 g/500 mL).

Working buffer: 500 uL of (a), 500 uL of (b), 100mL of (c), and 500 mL of (d), pH adjusted to 10.2 with HC1 and diluted to 500 mL.

(ii) Diethanolamine pH 9.5 (4% ethanol)

Diethanolamine 50 mM, MgCl₂ 1mM, ZnSO₄ 0.1 mM, 4% ethanol. Dissolved 2.5 gm diethanolamine in water, added ZnSO₄, and MgCl₂ as above, and 20mL of ethanol. Adjusted to pH 9.5 with 0.2M HC1, and diluted to 500mL.

(iii) Phosphate 0.2 M, pH 7.2

This was used as an inhibitor of the alkaline phosphatase reaction in the immunoassay. Prepared by mixing approximately 28 mL 0.2 M monobasic sodium phosphate with 72 ml 0.2 M dibasic sodium phosphate.

Alkaline phosphatase preparation

3mgs of Alkaline phosphatase (E.C. 3.1.3.1) as supplied by Boehringer was dissolved in 1 mL of Tris buffer to give a solution containing $1.56 \times 10^{-6}$ Moles of enzyme. This preparation was frozen in 100 uL aliquotes. Addition of 900 uL Tris on thawing gave 1mL, $10^{-7}$ M enzyme.

Constant potential electrochemistry

Experiments were performed using a Rank cell, and the carbon electrode, potential 300mV w.r.t. Ag/AgCl reference / counter electrode. The reaction volume 500uL, consisting of 50uL enzyme and 450uL of 2mM substrate in Tris buffer, baseline achieved using buffer alone. Current measured using Sycopel potentiostat and Gould BS-271 chart recorder.

Alternatively, a small two electrode cell (1 mm diameter working electrode, 4 mm diameter reference / counter electrode) linked to a portable meter (Automated Systems Laboratories, Milton Keynes, UK) was used. A configuration using a glassy carbon electrode with a stainless steel reference (Goodfellow, Metals, Cambridge, England) was also investigated.

The electrode comprised carbon block granules having a small particle size eg carbon black such as Vulcan XC-72 having a nominal particle size of 30 nm, the particle sizes ranging between 5 to 30 nm. The carbon black was platinised electrolytically giving about 10% w/w of uniform platinum coating in the form of crystallites 0.5 - 2.5 nm in diameter, substantially in the range 1.5 - 2.5 nm. The resultant material of surface area 200 $m^2g^{-1}$ was compounded with 50% w/w polytetrafluoroethylene and bonded to a 1 mm conducting carbon paper support. The 0.2 mm thickness of electrode material had a Pt loading of 0.5 mg $cm^{-2}$.

Substrates (phenolphthalein phosphate, 4-methyl umbelliferyl phosphate, 4-nitrophenyl phosphate and 1-naphthyl phosphate) at concentrations of 2mM in Tris buffer were incubated for 30 mins with standard alkaline phosphatase solutions, 50uL enzyme + 450uL buffered substrate, and then transferred to the electrochemical cell, where the current was measured at the carbon electrode at 300mV vs Ag/AgCl. A polycarbonate membrane 0.1um pore size was used to cover the electrode. The current (uA) was recorded after two minutes.

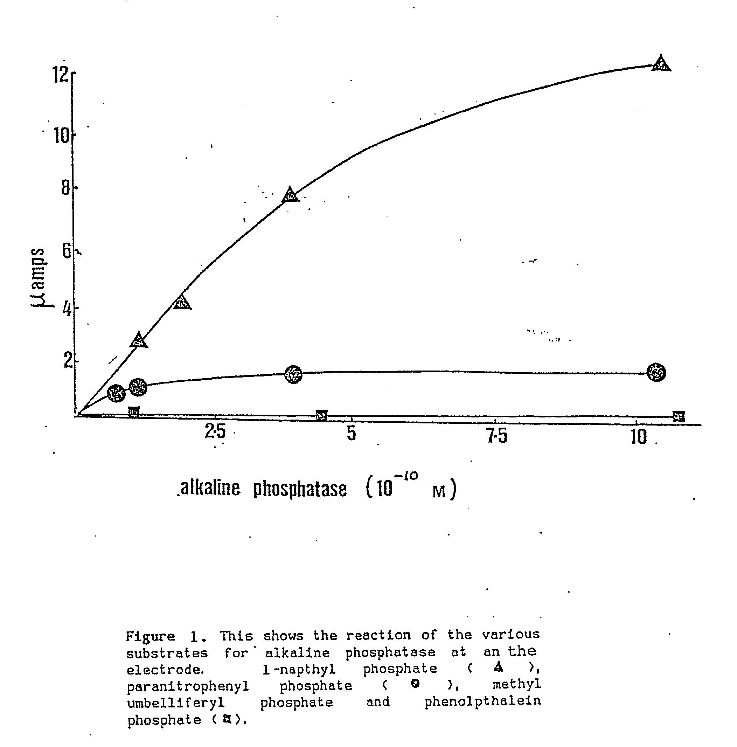

From the results (Table 1, Figure 1) it was apparent that 1-naphthyl phosphate was most advantageous. Other commonly used substrates gave poor responses at this electrode.

Alkaline phosphatase (1.56 x 10 $^{-10}$ M) was incubated with 2mM 1-naphthyl phosphate for varying times and then transferred to the electrochemical cell used above and the current recorded.

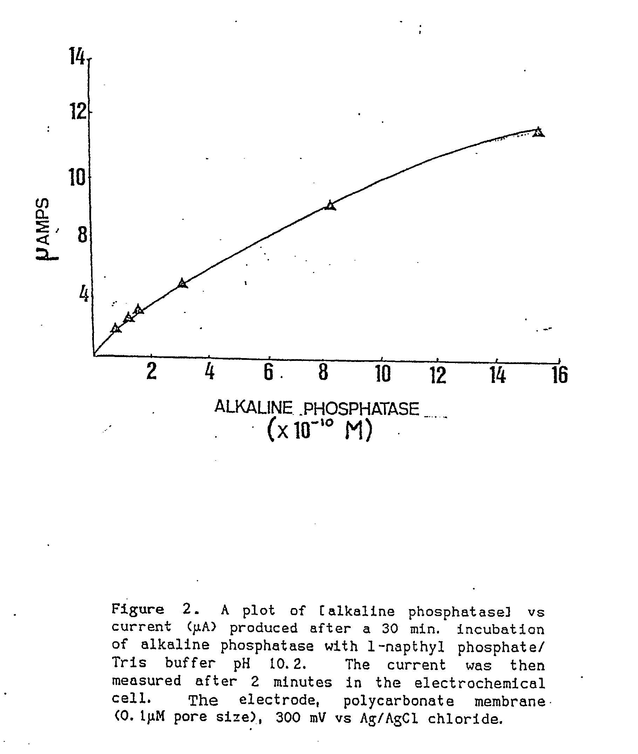

Standards containing different concentrations of alakaline phosphatase were produced, and incubated with 1-naphthyl phosphate under the conditions described above for thirty minutes, or the reaction mixture was transferred to the electrochemical cell and the current (uA) was recorded after two minutes in the cell. Figure 2 shows the relationship between alkaline phosphatase concentration and the current produced.

A range of alkaline phosphatase standards was produced as previously described and incubated with 1-naphthyl phosphate in Tris buffer for 15 minutes. The samples were then transferred to an electrochemical cell: AGE 300mV vs Ag/AgC1, stirred, polycarbonate membrane 0.1 uM working volume 500uL, and the current recorded.

The results were compared with results obtained from standards assayed at a glassy carbon electrode Ag/AgC1 reference. The electrode was swept between 0-500 mVolts and the peak current measured. Between measurements the electrode was cleaned by polishing with an aluminium oxide slurry on cotton wool followed by sonication for ten seconds.

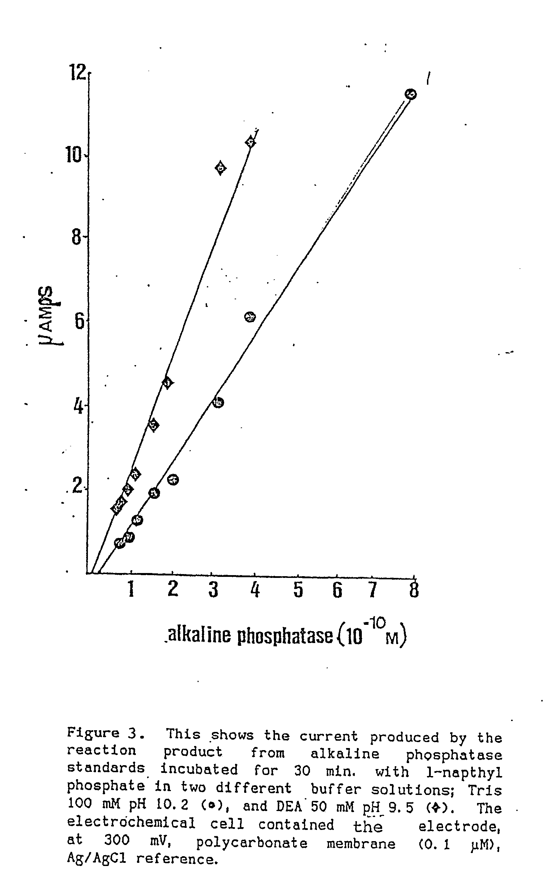

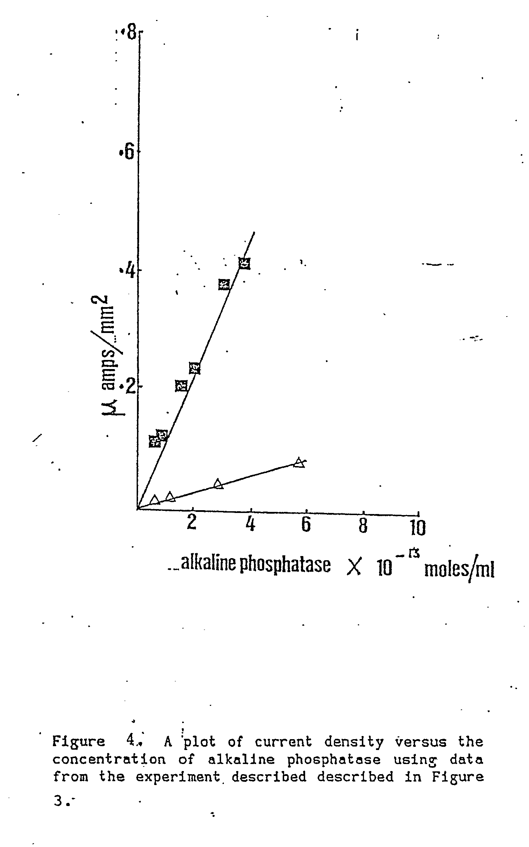

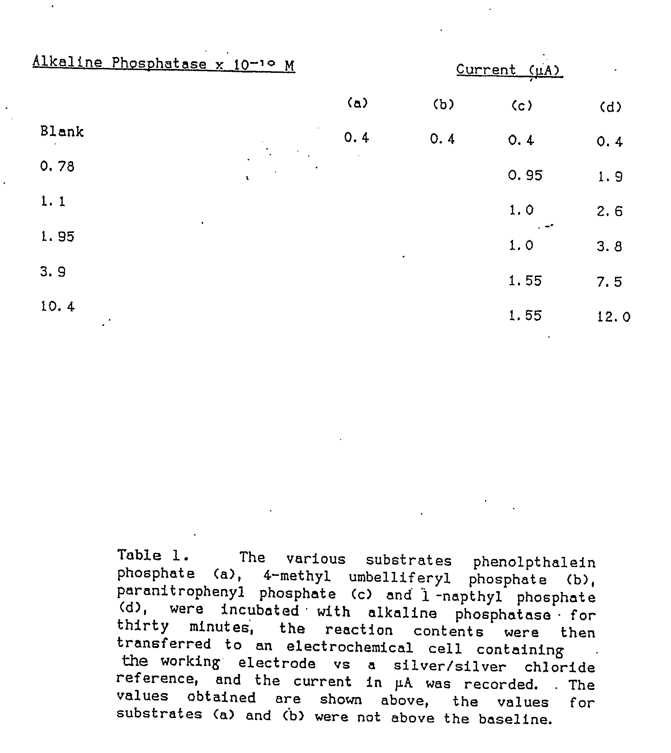

The results show (Figures 3 and 4) that the present invention is capable of producing much greater current responses than glassy carbon, the most commonly used electrode material in biochemical analysis.

4

Table 2.

| 5 minute incubation | |
| --- | --- |
| ALP x 10$^{-10}$M | $\mu$Amps (-blank) |
| 15.6 | 3.30 |
| 7.8 | 2.20 |
| 3.9 | 1.55 |
| 2.6 | 1.10 |
| 1.95 | 1.05 |
| 1.56 | 1.00 |
| 20 minute incubation | |
| ALP M | $\mu$Amps (-blank) |
| $10^{-8}$ | 32.3 |
| $10^{-9}$ | 6.8 |
| $10^{-10}$ | 1.0 |
| $10^{-11}$ | 0.2 |
| Results from electrochemical assays, involving alkaline phosphatase incubated with l-napthyl phosphate, currents recorded at 300 mVolts at an ACE electrode. | |

Table 3.

| 5 minute incubation | |
| --- | --- |
| ALP x $10^{-10}$ M | Absorbance ( 504 nm ) |
| 15.6 | 1.041 |
| 7.8 | 0.502 |
| 1.56 | 0.150 |
| 0.78 | 0.091 |
| 0.156 | 0.41 |
| 0.078 | 0.035 |
| 0.0156 | 0.036 |
| 20 minute incubation | |
| ALP (M) | Absorbance ( 504 nm ) |
| $10^{-11}$ | Over Range |
| $10^{-12}$ | 1.707 |
| $10^{-13}$ | 0.276 |
| $10^{-14}$ | 0.063 |
| $10^{-15}$ | 0.035 |
| $10^{-16}$ | 0.035 |
| The results of optical assays using alkaline phosphatase incubated with paranitrophenyl phosphate, measurement of absorbance at 405nm. | |

Tables 2 and 3 show the comparison between data obtained from an optical assay using 4-nitrophenyl phosphate and an electrochemical assay using 1-naphthyl phosphate when incubated with alkaline phosphatase for 5 minutes and for 20 minutes. For the electrochemical assay the range for five minutes was $10^{-11}$ M to $10^{-9}$ M (and possibly higher), the increased incubation did not appear to increase the sensitivity by an order or magnitude. In the optical assay the range for five minutes incubation was $10^{-11}$ M to $10^{-9}$M, and for twenty minutes incuvation $10^{-14}$ M to $10^{-12}$M. An increase in sensitivity at one end is matched by a loss of range at the top end. Sensitivity may be increased by using kinetic measurements.

1-Naphthyl phosphate gave comparative sensitivity to 4-nitrophenyl phosphate over a five minute incubation, the range being extended to enable measurement at higher concentrations.

The products 1-naphthol and 4-aminophenol are produced from the reactions between alkaline phosphatase and 1-1-naphthyl phosphate and 4-aminophenyl phosphate respectively. An experiment was performed to investigate the detection of these products at the electrodes.

A range of concentrations of 1-naphthol and 4-aminophenol were produced from a 2mM stock in Tris buffer, and the currents were measured after two minutes in the electrochemical cell. The cell contained an electrode at 300mV vs Ag/AgCl, covered by a polycarbonate membrane, pore size 0.1 uM.

An experiment was carried out to compare the current densities obtained from an arrangement in accordance with the present invention with an arrangement as disclosed by Frew et al (J Electroanal Chem 266 (1989) 309-316), particulate carbon electrodes constructed in accordance with US 4545382 having an area of 6 x 2mm being employed. The apparatus of the present invention afforded current densities approximately 50 times greater than the prior art as shown below.

| [Alkaline phosphatase] (x $10^{-10}$ M) | Current Density (uA $cm^{-2}$) | |
|---|---|---|
| | Invention | Prior Art |
| 4.0 | 255 | 4.6 |
| 10.0 | 636 | 10.0 |
| 20.0 | 1082 | 22.0 |

## Claims

1. A method of electrochemical alkaline phosphatase immunoassay characterised in comprising the steps of:
contacting alkaline phosphatase with 1-naphthyl phosphate, allowing the phosphatase to hydrolyse the 1-naphthyl phosphate to form 1-naphthol and detecting the electrochemical oxidation potential of said 1-naphthol using an electrode comprising particles of carbon having a particle size of 3 to 50nm the particles carrying a platinum group metal.

2. A method as claimed in Claim 1, wherein said particles are bonded using a polymeric resin.

3. A method as claimed in Claim 2, wherein the resin is a fluorocarbon resin.

4. A method as claimed in Claim 3, wherein the resin is polytetrafluoroethylene.

5. A method as claimed in any preceding claim, wherein said particles have a size of 5 to 30nm.

6. Alkaline phosphate immunoassay apparatus including at least one electrochemical cell containing a reference electrode and characterised in further comprising an electrode comprising carbon particles having a particle size of 3 to 50nm the particle carrying a platinum group metal.

7. Apparatus as claimed in Claim 6, wherein said particles are bonded using a polymeric resin.

8. Apparatus as claimed in Claim 7, wherein the resin is polytetrafluoroethylene.

9. Apparatus as claimed in any of Claims 6 to 8 wherein the carbon particles have a particle size of 5 to 30nm.

10. Alkaline phosphate immunoassay apparatus comprising a printed circuit board carrying an array of cells as claimed in any of claims 6 to 9.

Figure 1. This shows the reaction of the various substrates for alkaline phosphatase at an the electrode. 1-napthyl phosphate ( ▲ ), paranitrophenyl phosphate ( ● ), methyl umbelliferyl phosphate and phenolpthalein phosphate ( ■ ).

Figure 2. A plot of [alkaline phosphatase] vs current (µA) produced after a 30 min. incubation of alkaline phosphatase with 1-napthyl phosphate/ Tris buffer pH 10.2. The current was then measured after 2 minutes in the electrochemical cell. The electrode, polycarbonate membrane (0.1µM pore size), 300 mV vs Ag/AgCl chloride.

Figure 3. This shows the current produced by the reaction product from alkaline phosphatase standards incubated for 30 min. with 1-napthyl phosphate in two different buffer solutions; Tris 100 mM pH 10.2 (•), and DEA 50 mM pH 9.5 (◆). The electrochemical cell contained the electrode, at 300 mV, polycarbonate membrane (0.1 μM), Ag/AgCl reference.

Figure 4. A plot of current density versus the concentration of alkaline phosphatase using data from the experiment described described in Figure 3.

| Alkaline Phosphatase x $10^{-10}$ M | Current (μA) | | | |
|---|---|---|---|---|
| | (a) | (b) | (c) | (d) |
| Blank | 0.4 | 0.4 | 0.4 | 0.4 |
| 0.78 | | | 0.95 | 1.9 |
| 1.1 | | | 1.0 | 2.6 |
| 1.95 | | | 1.0 | 3.8 |
| 3.9 | | | 1.55 | 7.5 |
| 10.4 | | | 1.55 | 12.0 |

Table 1. The various substrates phenolpthalein phosphate (a), 4-methyl umbelliferyl phosphate (b), paranitrophenyl phosphate (c) and 1-napthyl phosphate (d), were incubated with alkaline phosphatase for thirty minutes, the reaction contents were then transferred to an electrochemical cell containing the working electrode vs a silver/silver chloride reference, and the current in μA was recorded. The values obtained are shown above, the values for substrates (a) and (b) were not above the baseline.